# EUROPEAN PATENT SPECIFICATION

(11) **EP 1 486 201 B1**
(45) Date of publication and mention of the grant of the patent: **24.12.2008**
(21) Application number: 04013502.2
(22) Date of filing: 08.06.2004
(51) Int. Cl.: A61Q 19/02, A61K 8/99

(54) **Use of a cosmetic material comprising extract from Schizosaccharomyces for whitening of the skin**
Verwendung eines kosmetischen Wirkstoffs mit Extrakten von Schizosaccharomyces zur Aufhellung der Haut
Utilisation d'une composition cosmétique comprenant des extraits de Schizosaccharomyces pour éclaircir la peau

(30) Priority: 09.06.2003 JP 2003163513
(43) Date of publication of application: 15.12.2004
(73) Proprietor: NIHON KOLMAR CO., LTD., Chuo-ku, Osaka (JP)
(72) Inventor: Tanaka, Katsumasa, Kashiwara-shi Osaka (JP); Nagai, Tsutomu, Kashiwara-shi Osaka (JP)
(74) Representative: Grünecker, Kinkeldey, Stockmair & Schwanhäusser Anwaltssozietät

(56) References cited:
- EP-A- 0 208 805
- EP-A- 0 819 762
- WO-A-20/04012650
- WO-A-20/04045574
- DE-A- 4 138 192
- FR-A- 1 261 173
- FR-A- 2 809 308
- US-A- 4 980 151
- US-B1- 6 368 594
- DATABASE WPI Week 1989 Derwent Publications Ltd., London, GB; AN 1989-223006 XP002297208 NN: "Accumulation of glutathione in fungus body - by adding composite aminoacid to culture medium at start of culture, then at later stage" & JP 01 148181 A (JGC CORP.) 9 June 1989 (1989-06-09)

## Description

This invention relates to the use of a cosmetic material.

Melanin, a dark brown pigment, is believed to be causes of pigmentation, which tends to develop after sun-bathing or where acnes were, hepatic patches, from which women in their menopausal years frequently suffer, and senile pigmetary patches, which develop among older people.

When human skin is exposed to external stimuli such as ultraviolet rays, tyrosinase that exists in melanocytes of the skin is activated, and tyrosine, an amino acid, is oxidized, so that melanin is produced. This suggests that one can maintain white skin if she or he can successfully inhibit the activity of tyrosinase, which is, as mentioned, believed to play a key role in the production of melanin. In view of this finding, cosmetic materials that contain tyrosinase activity inhibiting ingredients are now available.

It has, however, been found out that when they are actually applied to human skin, none of them can sufficiently inhibit the production of melanin in B16 cultured melanoma cells or achieve apparent thinning of pigments that have already deposited.

It has further been found out that in order for such a cosmetic material to reliably exhibit its expected function, i.e. whitening of the skin, the cosmetic material has to have more than just the tyrosinase inhibiting function and the melanin production inhibiting function. It has to be further capable of erasing free radicals, thereby inhibiting pigmentation, and activating epidermal cells, thereby speeding up excretion of melanin.

Among such conventional cosmetic materials, the one disclosed in JP patent publication 7-10734 contains yeast, particularly a fermented culture solution of a yeast of the genus *Saccharomyces* as an active ingredient to increase whiteness of the skin.

JP patent publication 8-217658 discloses a ceramide synthesis promoter that activates the epidermic cells in the superficial layer of the skin to cause them to synthesize ceramide, thereby improving the skin barrier function. JP patent publication 2000-109408 discloses a skin whitening cosmetic material containing a glucoside such as raspberry ketone β -D glucoside and an yeast extract, or a fungus culture containing such a glucoce and yeast extract.

US 6,368,594 B describes the isolation and purification of a polypeptide fragment from Schizosaccharomyces pombe, a truncated form of UVDE, which exhibits superior stability and enzymatic activity in repairing DNA damage than purified full-length UVDE.

DE 4138192 A describes an agent on herbal basis for renewing the skin of fresh burned injuries, for cleanising the skin, for combating varicose veins and for treating swellings. The agent is obtained by fermenting sugared green tee with Kombucha which consists *inter alia* of Schizosaccharomyces pombe.

FR 1261173 A relates to the use of Kombucha for amending nutrition and cosmetic products.

WO 2004/012650 A describes cosmetic or dermopharmaceutical compositions which contain Kombucha. Said compositions can be used for the care of the skin, mucosae and skin appendages, and for preventing the signs of endogenous and/or exogeneous aging.

US 4,980,151 describes an anticariogenic or antiperiodontitic agent containing, as an active component, yeast cells and/or water-soluble extracts of yeasts belonging *inter alia* to the genus Schizosaccharomyces having antibacterial activity against Streptococcus mutans or Bacteroides gingivalis.

EP 0819762 A describes a process for the isolation of glucane from yeast wherein the yeast is treated with an alkaline surfactant-containing solutaion and obtained solid glucane containing residue is contacted with an alcohol wherein the glucane is insoluble. The glucane can be used in, for example, cosmetic compositions for the treatment and prophylaxis of skin diseases.

FR 2809308 A discloses cosmetic and dermatopharmaceutical compositions comprising heat shock proteins (HSP) isolated from yeast, which may be inter alia Schizosaccharomyces, which act against stress resulting from UV radiation or oxidative agents.

Any of the abovementioned conventional cosmetic materials contains a yeast extract alone or a yeast extract and a glucoside, but cannot sufficiently inhibit the tyrosinase activity and melanin production to such an extent that white skin can be reliably maintained.

An object of the present invention is to provide a cosmetic material which not only inhibits the tyrosinase activity and melanin production but also erases free radicals and activates cells, thereby reliably whitening the skin.

Said object is achieved by the subject matter set forth in claims 1 to 3.

In genetic analysis of yeasts which proliferate by splitting, the inventors of the present invention have discovered that these yeasts are fission cells which proliferate more like human cells than do budding yeasts. This finding led the inventors to think that these yeasts may contain ingredients having far higher activities than those contained in conventional yeast extracts. In view of these findings, the inventors specifically examined the effects of cell extracts and spore extracts of a particular fission yeast that belongs to the genus *Schizosaccharomyces* on human skin in comparison with other yeasts. It has been found out that only this particular yeast has all of the abovementioned properties. The inventors have therefore decided to use this particular yeast to prepare the cosmetic material according to this invention.

As will be apparent from the test results in the following description of the invention, an extract in an aqueous solvent of a yeast of the genus *Schizosaccharomyces* activates cells, i.e. continuously produces new cells by splitting fibloblasts, and also suppresses production of melanin. Thus, the cosmetic material according to this invention, which contains this particular yeast extract, can maintain white skin due to the synergetic effects of the above properties.

In order for the cosmetic material used according to this invention to reliably and fully reveal its expected functions, as the yeast of the genus *Schizosaccharomyces, Schizosaccharomyces pombe* is preferably selected.

The content of the yeast extract in the cosmetic material is preferably 0.05 to 10 wt%.

### BRIEF DESCRIPTION OF THE DRAWINGS

Other features and objects of the present invention will become apparent from the following description and the accompanying drawings, in which:
Fig. 1 is a graph showing how the melanin content in the skin to which different lotions were applied changes with time; and
Fig. 2 is a graph showing how the moisture content in the keratin of the skin to which various lotions were applied changes with time.

### DETAILED DESCRIPTION OF THE PREFERRED EMBODIMENT

The yeast used in the present invention is a yeast of the genus *Schizosaccharomyces,* which is single-cell ascomycetous eukaryotes. Each cell thereof uniformly fissions into two cells just like animal cells. Therefore, this type of yeasts are hereinafter called "fission yeasts". A typical yeast of the genus *Schizosaccharomyces* is *Schizosaccharomyces pombe.*
The word "schizo" means "split".

*Schizosaccharomyces pombe* has been used for foods from old times. It has been well-known world-widely since it was isolated from "Millet beer" (*"pombe"* in Swahili) made in East Africa in 1890s. Such fission yeasts are widely used for the production of grape juice, palm wine, sugarcane syrup and bread, and brewing beer. Fission yeasts are easily commercially available and their safety has been confirmed by various authorities.

In a nutritious environment, fission yeasts multiply due to somatic mitosis. In an ill-nourished environment and in the presence of sex pheromone, they perform sexual reproduction in which spores are formed after mating.

A culture medium used in culturing such fission yeasts may be an ordinary one used for yeasts. If it is desired to obtain cells and spores of the yeasts separately from each other, synthetic medium is preferably used. To a medium for forming spores, no nitrogen source should be added. Culture conditions, such as temperature and time, may be substantially the same as conditions under which ordinary yeasts are cultured.

Cells and spores of fission yeasts are extracted in the following manner:

Fungi are collected from the cultured yeasts, sufficiently rinsed with e.g. purified water, and suspended in purified water, the amount of which is 1/10 the amount of the medium. 2.5 mg of a cell wall decomposing enzyme is added to every milliliter of the fungi and the fungi were further cultured for four days at 37 degrees Celsius to let the fungi react with the enzyme. The culture period (days) may be longer or shorter than four days. The fungi should be cultured until a steady state is reached, which can be determined by checking e.g. the temperature of the medium.

When a steady state is reached, the enzyme is deactivated, and the fungi are centrifuged and filtered. Alternatively, to the rinsed and thus wet fungi, purified water in an amount of 10-100 times the amount of the fungi is added to allow the fungi to self-digest for about 24-72 hours at 35-45 degree Celsius, and then the fungi are freeze-dried or condensed under reduced pressure. An aqueous solvent such as water, ethanol, butylene glycol, propylene glycol or glycerin, or a mix of two or more of them is added to the wet fungi in an amount 10-100 times the amount of the fungi, and then the fungi are centrifuged and filtered.

Further alternatively, purified water is added to the rinsed and thus wet fungi in an amount 10-100 times the amount of the fungi, and about 200 to 500 thousand units of protease, an enzyme, is added per kilogram of the fungi to allow the fungi to be decomposed by the enzyme at an enzyme activated temperature around the clock.

The enzyme is then deactivated, and with an aqueous solvent added or not added, the fungi are centrifuged and filtered. Further alternatively, 1-5% hydrochloric acid is added to the rinsed and thus wet fungi in an amount 10 times the fungi, and the fungi are hydrolyzed while stirring at 40-60 degrees Celsius for 3-8 hours, neutralized using alkalis, and treated in the same manner as above. After neutralizing, they may be desalted by e.g. dialysis.

The thus obtained fission yeast extract used in the present invention is added by 0.05-10 wt% (preferably 2-5 wt%) to a cosmetic material as its active ingredient. Such a cosmetic material exhibits the functions of whitening the skin, erasing free radicals, activating cells.

The aqueous fission yeast extract of the present invention can be used with bases, additives and other agents used in cosmetics to prepare various cosmetic items.

The aqueous fission yeast extract used in the invention is typically mixed with an animal, vegetable or mineral oil, or any other oil or fat including ester oil, wax, higher alcohol, a fatty acid, silicon oil, or lipid phosphoric acid.

Surface-active agents usable with the fission yeast extract used in the present invention include anionic ones, cationic ones, amphoteric ones and non-ionic ones. If so desired or necessary, a cosmetic material containing the fission yeast extract used in the present invention may further contain ultraviolet absorbers such as p-aminobenzoic acid, anthranil derivatives, benzotriazole derivatives, tetrazole derivatives, imidazoline derivatives, pyrimidine derivatives, dioxane derivatives, camphor derivatives, furan derivatives, pyrone derivatives, nucleic acid derivatives, allantoin derivatives, nicotinic acid derivatives, shikonin and vitamin B6 derivatives; anti-oxidants such as ascorbic acid and its salts, stearates, tocopherol and its ester derivativces, nordihydroguaiaretic acid, butylhydroxytoluene (BHT), butylhydroxyanisole (BHA), parahydroxyanisole and propyl gallate; thickening agents such as hydroxyethyl cellulose, methyl cellulose, ethyl cellulose, carboxymethyl cellulose, carboxyethyl cellulose, gum arabic, polyvinyl alcohol, polyvinyl pyrrolidone, polyvinylmethacrylate, polyacrylate, carboxyvinyl polymers, carrageenan, pectin, alginic acid and its salts, casein and gelatin; humectants such as glycerine, propylene glycol, 1,3-butylene glycol, hyaluronic acid and its salts, polyethylene glycol, chondroitin sulfate and its salts, water-soluble chitin or chitosan derivatives and sodium lactate; and other components and substances including lower alcohols, polyatomic alcohols, water-soluble polymers, pH adjusters, chelating agents, antiseptics, antibacterial agents, perfumes, colorants, refrigerants, stabilizers, animal and vegetable extracts, animal and vegetable proteins and their decomposition products, polysaccharides of animal and vegetable origin and their decomposition products, bloodstream promoters, antiphlogistics, anti-inflammatories, cell activators, vitamins, amino acids and their salts, caryoplasm dissolving agents, astringents, wound healers, foaming agents and deodorants.

The cosmetic material of this invention is used as a cosmetic or a non-prescription drug to prevent or improve pigmentation and activate skin cells. It may be in the form of cream, ointment, beauty wash, toilet water, packs, bathwater additives or makeups, or may be of any other form.

### [Examples]

### (Culture Example 1 of fission yeast)

Culture media 1 and 2 having the following compositions were prepared. In the medium 1, one platinum loop-full of fission yeast was put and cultured for three days at 30 degrees Celsius. The culture solution was centrifuged to collect fungi. The entire fungi were suspended in an equal amount of the medium 2, and cultured for five days. The culture solution thus obtained was centrifuged to collect fungi, which were then sufficiently rinsed with purified water.

| | | |
|---|---|---|
| <Medium 1> | Glucose | 30 g |
| | Yeast extract | 5 g |
| <Medium 2> | Glucose | 10 g |
| | Potassium dihydrogenphosphate | 0.25 g |
| | Calcium chloride | 0.1 g |
| | Boric acid | 0.5 mg |
| | Copper sulfate | 40 µg |
| | Potassium iodide | 0.1 mg |
| | Iron chloride | 0.2 mg |
| | Manganese sulfate | 0.4 mg |
| | Molybdic acid | 0.16 mg |
| | Zinc sulfate | 0.4 mg |
| | Biotin | 1 µg |
| | Calcium pantothenate | 1 mg |
| | Nicotinic acid | 10 mg |
| | Inositol | 10 mg |

### (Culture Example 2 of fission yeast)

Fission yeast was put (by one platinum loop full) in the same culture medium 1 as used in Culture Example 1 and cultured for 10 days at 30 degrees Celsius. The culture solution obtained was centrifuged to collect fungi, which were then sufficiently rinsed with purified water.

### (Fission Yeast Extract Example 1)

0.1 to 0.1 kg of the fission yeast obtained in Culture Example 1 was suspended in distilled water in an amount 1/10 of culture volume. Lysing enzymes (lot No. 69H1557; made by SIGMA) was added to the suspension, and the suspension was left for four days at 30 degrees Celsius. Then, the suspension was treated with ultrasonics until the total amount was 100 ml. This solution was heated for an hour at 90 degrees Celsius to deactivate the enzymes, and filtered using a filtering assistant, filter paper and a membrane filter.

### (Fission Yeast Extract Example 2)

The fission yeast obtained in Culture Example 2 was suspended in distilled water in an amount 1/10 of culture volume. Lysing enzymes (lot No. 69H1557; made by SIGMA) was added to the suspension, and the suspension was left for four days at 30 degrees Celsius. Then, the suspension was treated with ultrasonics until the total amount was 100 ml. This solution was heated for an hour at 90 degrees Celsius to deactivate the enzymes, and filtered using a filtering assistant, filter paper and a membrane filter.

### [Comparative Extract Examples 1 and 2] (Budding yeast extracts)

Comparative Examples 1 and 2 are both yeast extracts of *Saccharomyces cerevisiae,* which is a budding yeast, made by different manufacturers.

### <Test for determining the ability of each extract example to inhibit melanin synthesis>

A test was conducted to determine how effectively each of Fission Yeast Extract Examples 1 and 2 (of the invention) and Comparative Budding Yeast Extract Examples 1 and 2 can inihibit the synthesis of melanin in melanoma cells.

The melanoma cells used in the test were prepared by adding B16 mouse melanoma cells to 10% FBS-containing MEM mediums and culturing the cells at 37 degrees Celsius in an atmosphere containing 5% of CO₂. Specifically, 1T: × 5 B16 melanoma cells were planted in 60 mm plastic Petri dishes and cultured for 24 hours. Then, the mediums were replaced with new ones, and each extract example was added by 2% to one of the mediums. Three days after adding the extract examples, the cells were recovered by trypsyn treatment and dissolved by heating in 1N NaOH, 10% DMSO solution to measure the light absorbance at 475 nm. The melanin synthesis rates when the extract samples were added were calculated which were the rates relative to the melanin synthesis rate when the cells were not treated. The melanin synthesis inhibition rates (%) shown in Table 1 are therefore the melanin synthesis rate in non-treated cells minus the melanin synthesis rates when the respective extract examples were added.

**Table 1**

| Specimen | (%) |
|---|---|
| Fission Yeast Extract Example 1 | 62 |
| Fission Yeast Extract Example 2 | 66 |
| Comparative Extract Example 1 (Commercial Product) | 21 |
| Comparative Extract Example 2 (Commercial Product) | 51 |

Table 1 clearly shows that the fission yeast Extract Examples 1 and 2 have far higher ability to inhibit the synthesis of melanin, which is a leading cause of stains and freckles, than Comparative Extract Examples 1 and 2, which are commercially available budding yeast extracts.

### <Test for determining the ability to activate (grow) cells>

The test was conducted for Extract Examples 1 and 2 (of the invention) and Comparative Extract Examples 1 and 2 on normal human skin fibroblasts NB1RGB cultured in a 5% FBS-containing MEM medium at 37 degrees Celsius in an atmosphere containing 5% CO₂. Specifically, 0.5 x 10⁴ normal human skin fibroblasts in a 5% FBS-containing MEM medium were planted in 96-well microplates, and cultured for 24 hours. The culture solutions thus obtained were then put in 0.5% FBS-containing MEM mediums each containing one of extract examples of the invention and comparative extract examples, and cultured for another nine days. The culture solutions thus obtained were further put in 0.5% FBS-containing MEM mediums containing neutral red (NR) by 50 micrograms per milliliter, and cultured for additional two hours. The culture solutions were then discarded, and the microplates were rinsed with a 1% CaCl₂, 1% formalin aqueous solution. To the thus rinsed microplates, an aqueous solution of 1% acetic acid and 50% ethanol was added to extract the NR, in which the fibroblasts were trapped. The absorbance at 570 nm of the microplates were then measured by means of a microplate reader. The results of measurement are shown in Table 2.

**Table 2**

| Specimen/Cell Growth Rate (%) | Sample Concentration (%) | | | | | | |
|---|---|---|---|---|---|---|---|
| | 0 | 0.3125 | 0.625 | 1.25 | 2.50 | 5.00 | 10.0 |
| Extract Example 1 (Fission Yeast Extract Example 1) | 100.00 | 123.65 | 141.31 | 162.74 | 194.02 | 236.00 | 278.29 |
| Comparative Extract Example 1 | 100.00 | 135.33 | 146.67 | 146.67 | 186.67 | 210.00 | 166.67 |
| Comparative Extract Example 2 | 100.00 | 121.33 | 133.33 | 163.33 | 183.33 | 193.33 | 166.67 |

Table 2 clearly shows that the fission yeast extracts has a higher ability to accelerate the growth of cells, i.e. activate cells than the Comparative Extract Examples 1 and 2. Specifically, they grow cornified cells and fibroblasts of the skin more efficiently than Comparative Extract Examples 1 and 2, thereby improving the skin condition.

### <Preparing lotions and determining their effects>

Four kinds of lotions were prepared, that is, Example 1, which contained the fission yeast extract of Extract Example 1 at the rate shown in Table 3, Comparative Examples 1 and 2, which contained the Comparative Extract Examples 1 and 2, respectively, at the same rate as the fission yeast extract in Example 1, and Comparative Example 3, which is a blank, i.e. contains no yeast extracts. Suitable amounts of antiseptics and pH adjustors were added to each of the lotions.

The four kinds of lotions thus prepared were separately applied to the skin (facial skin) of a total of 20 subjects (consisting of 10 male adults and 10 female adults) for two months, twice a day, in the morning and evening. At the end of the two-month period, each subject answered the five questions shown in Table 4 by selecting one of five levels 1 to 5. The average scores for the respective examples for the respective questions are shown in Table 3.

**Table 3**

| Component (wt%) | Example | Comparative Example | | |
|---|---|---|---|---|
| | 1 | 1 | 2 | 3 |
| Fission Yeast Extract | 2.5 | 0 | 0 | 0 |
| Purified Water | 0 | 0 | 0 | 2.5 |
| Conventional Yeast Extract 1 | 0 | 2.5 | 0 | 0 |
| Conventional Yeast Extract 2 | 0 | 0 | 2.5 | 0 |
| Glycerin | 10 | 10 | 10 | 10 |
| Maltitol | 3 | 3 | 3 | 3 |
| Ethanol | 5 | 5 | 5 | 5 |
| Purified Water | balance | balance | balance | Balance |

| Test Item | | | | |
|---|---|---|---|---|
| 1. Dampness | 4.3 | 3.1 | 2.9 | 2.3 |
| 2. Brightness | 4.5 | 2.9 | 3.2 | 1.8 |
| 3. Roughness | 4.7 | 3.5 | 3.6 | 2 |
| 4. Wrinkles | 4.8 | 3.7 | 3.6 | 1.5 |
| 5. Overall Score | 4.7 | 3.5 | 3.5 | 2.4 |

**Table 4**

| Score | Has dampness improved or worsened? | Has brightness improved or worsened? | Has roughness improved or worsened? | Has wrinkles Has wrinkles reduced? |
|---|---|---|---|---|
| 5 | Improved very much | Improved very much | Improved very much | Yes, very much |
| 4 | Moderately improved | Improved | Improved | Yes |
| 3 | No change | Moderately improved | Moderately improved | Yes, slightly |
| 2 | Slightly worsened | No change | No change | No change |
| 1 | Worsened very much | Slightly worsened | Slightly worsened | Slightly increased |

### <Test for determining the skin whitening effect after the respective lotions have been applied>

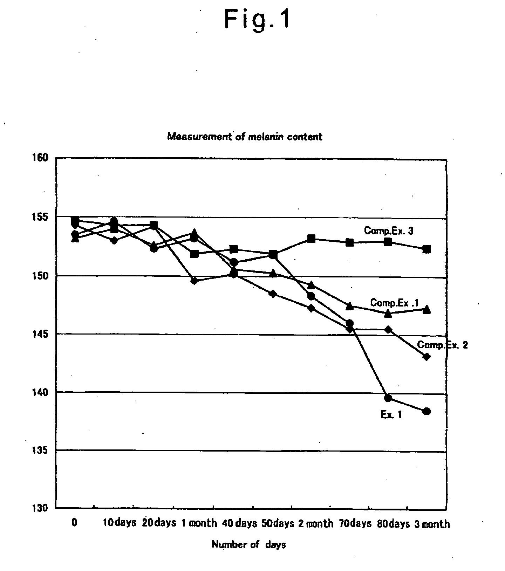

The lotions shown in Table 3 were separately applied to the skin (facial skin) of a total of 20 subjects (10 each male and female adults) for the period of three months, twice every day, in the morning and evening. During and at the end of the three-month period, the amount of melanin in the skin was measured for each subject using a whitening effect tester (MEXAMETER MX18) made by CK. The results of measurement are shown in Fig. 1.

As is apparent from Fig. 1, after 70^{th} day until the end of the three-month period, the amount of melanin in the skin to which the extract containing Example 1 was applied dropped far more sharply than the amount of melanin in the skin to which lotions containing Comparative Examples were used.

### <Change in the moisture content in skin keratin when lotions were applied>

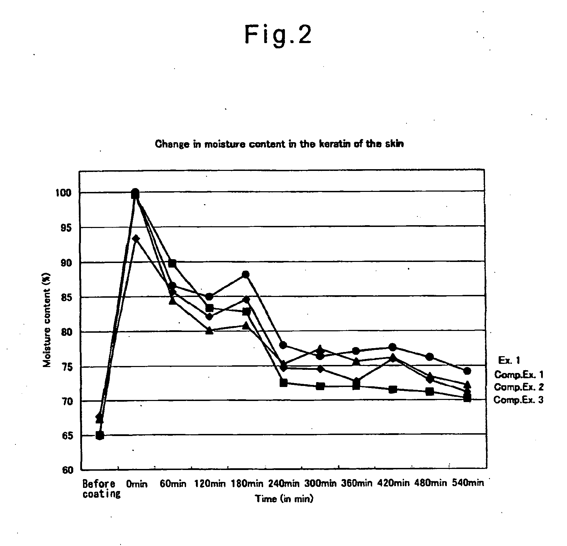

Lotions containing Example 1 and Comparative Examples 1-3 were applied to human skin and the moisture contents in the skin keratin were measured using a moisture content tester made by CK at predetermined time intervals until 540 minutes had passed after application of the lotions. The results of measurement are shown in Fig. 2.

As is apparent from Fig. 2, after 120 minutes from the start of the experiment, the lotion containing Example 1 maintained higher moisture contents of skin keratin than the lotions containing Comparative Examples 1-3.

The cosmetic material used in the present invention, which contains an extract of an aqueous solution of *Schizosaccharomyces,* a fission yeast, as an active ingredient, can far more effectively suppress production of melanin in cells, thereby maintaining white skin, and also can activate cells far more effectively than conventional cosmetic materials such as those containing budding yeast or its extracts.

## Claims

1. Use of a cosmetic material comprising an extract in aqueous solvent of a yeast of the genus Schizosaccharomyces as the active ingredient for whitening the skin.

2. The use as claimed in claim 1, wherein said yeast of the genus Schizosaccharomyces is Schizosaccharomyces pombe.

3. The use as claimed in claim 1 or 2, wherein the content of said active ingredient is 0.05 to 10 wt%.

## Patentansprüche

1. Verwendung eines kosmetischen Materials, umfassend einen Extrakt in wässrigem Lösungsmittel aus einer Hefe der Gattung Schizosaccharomyces als den Wirkstoff, zum Bleichen der Haut.

2. Verwendung wie in Anspruch 1 beansprucht, worin die Hefe der Gattung Schizosaccharomyces Schizosaccharomyce pombe ist.

3. Verwendung wie in Anspruch 1 oder 2 beansprucht, worin der Gehalt des Wirkstoffs 0,05 bis 10 Gew.-% ist.

## Revendications

1. Utilisation d'un matériau cosmétique comprenant un extrait dans un solvant aqueux d'une levure du genre Schizosaccharomyces comme ingrédient actif pour éclaircir la peau.

2. Utilisation selon la revendication 1, où ladite levure du genre Schizosaccharomyces est du Schizosaccharomyces pombe.

3. Utilisation selon la revendication 1 ou 2, où le contenu dudit ingrédient actif est de 0,05 à 10% en poids.
